# EUROPÄISCHE PATENTSCHRIFT

(11) **EP 1 560 605 B1**
(45) Veröffentlichungstag und Bekanntmachung des Hinweises auf die Patenterteilung: **28.03.2012**
(21) Anmeldenummer: 03767528.7
(22) Anmeldetag: 11.11.2003
(51) Int. Cl.: A61L 9/04, A61L 9/012, A61L 9/014, A61L 9/12, B01D 53/04

(54) **VERFAHREN ZUM DESODORIEREN VON GROSSFLÄCHIGEN ANLAGEN**
METHOD FOR DEODORISING LARGE-SCALE PLANTS
PROCEDE POUR DESODORISER DES INSTALLATIONS DE GRANDE SURFACE

(30) Priorität: 15.11.2002 DE 10253257
(43) Veröffentlichungstag der Anmeldung: 10.08.2005
(73) Patentinhaber: Air & D- Sarl, 67190 Rosheim (FR)
(72) Erfinder: WUEST, Robert, F-67190 Rosheim (FR)
(74) Vertreter: Ziegler, Stefan Michael
(86) Internationale Anmeldenummer: PCT/EP2003/012553
(87) Internationale Veröffentlichungsnummer: WO 2004/045657

(56) Entgegenhaltungen:
- EP-A- 1 334 736
- WO-A-01/78794
- WO-A-96/05870
- DE-A- 19 711 809
- DE-U- 20 118 329
- GB-A- 2 324 963
- US-A- 4 511 552

## Beschreibung

Die Erfindung betrifft ein Verfahren zum Desodorieren von großflächigen Anlagen, in denen übelriechende feste oder flüssige Stoffe offen gelagert sind, durch Behandeln der verunreinigten Luft über der Oberfläche der übelriechenden Stoffe mit aktiven Agentien, die mit den in der Luft enthaltenen übelriechenden Substanzen reagieren.

Großflächige Anlagen, in denen übelriechende Stoffe gelagert sind, sind zum Beispiel:
- Klärbecken, die eine schräg abfallende Einfassung aufweisen und mehrere hundert Quadratmeter groß sein können. Sie sind meist mit Industrieabwässem, die größere Mengen Feststoffe enthalten, gefüllt.
- Kläranlagen mit runden Becken, in denen Haushalt- und Industrieabwässer, sowie Abwässer aus Tierverwertungsanstalten gereinigt werden.
- Kompostieranlagen für Haus- und Gartenabfälle sowie für Industrieschlamm.
- Deponien, auf denen Haushalt- und Industrieabfälle gelagert sind.
- Müllsortieranlagen.
- Abwasserstapelbecken, z. B. in der Zuckerindustrie, Agrarindustrie und chemischen Industrie.
- Hafenbecken und offene Kanäle, insbesondere Abwasserkanäle.

Die Luft über der Oberfläche dieser Anlagen ist mit übelriechenden Gasen, wie z. B. Ammoniak, Aminen und Schwefelverbindungen geschwängert. Um das Geruchsproblem und die Umweltbelastung sowohl in der Nähe als auch in der weiteren Umgebung in den Griff zu bekommen, müssen aufwendige Maßnahmen, wie Abdeckung, Lagerung in geschlossenen Räumen, Einbau von Kaminen und Absauganlagen ergriffen werden, die aber meist unbefriedigend sind.
Die WO 01/78794 A2 beschreibt ein wasserfreies Gelelement zum Desodorieren von Luft oder geschlossenen Räumen, welches hergestellt wird durch Vernetzen eines funktionalisierten flüssigen Polymeren mit einem Vemetzer in Gegenwart des Desodorierungsmittels. Das Gelelement kann auf einen Rost aufgelegt werden, die in ein Gehäuse eingebracht sind, welches in Kontakt mit der Umgebungsluft steht. Von einer gezielten Freisetzung der Desodorierungsmittels durch einen Luftstrom ist nicht die Rede. Mit den entsprechenden Einrichtungen können daher keine großflächigen Anlagen desodoriert werden.
Die GB-A 2 324 963 beschreibt ein Verfahren zum Emittieren von aromatischen, therapeutischen oder antiseptischen Dämpfen in einen Wohnraum durch einen Aromaspender, bei dem ein faserartiges absorbierendes Material, welches ein flüssiges Öl enthält, zwischen zwei Platten so eingezwängt ist, dass die Außenkante des absorbierenden Materials zwischen den Außenkanten der Platten zu liegen kommt, und wobei die Platten einem zirkulierenden Luftstrom ausgesetzt werden.
Die DE-A 197 11 809 betrifft einen Duftspender aus Gummigranulat oder einem Granulat-Fasergemisch, dem Duft- oder Aromastoffe zugegeben werden.
Die DE-C 37 26 636 betrifft Desodorantien auf Basis von Zink-Ricinoleat, das u.a. zur Geruchsbeseitigung in Kläranlagen und Mülldeponien angewandt werden kann.
Es besteht daher das Bedürfnis nach einer effizienten und schnell wirkenden Beseitigung oder zumindest starker Verminderung dieser Geruchsbelastung.

Die daraus resultierende Aufgabe wird durch das erfindungsgemäße Verfahren gelöst. Gegenstand der Erfindung ist demzufolge ein Verfahren zum Desodorieren der genannten großflächigen Anlagen gemäß Anspruch 1.

Geeignete Matrix-Polymere sind vernetzte, hydrophile Gruppen enthaltende Polykondensate und vernetzte (Meth-)Acrylat-Polymere.

Bevorzugt sind Kondensationsprodukte aus einem maleinisierten oder epoxidierten Polymeren und einem Vernetzer, vorzugsweise einem Polyamin. Geeignete Polymere sind z. B. Umsetzungsprodukte von Polydienen, wie Polybutadien, Polydecadien und Soyabohnenöl mit Maleinsäureanhydrid, ferner Copolymere von Olefinen, wie Ethylen mit Maleinsäureanhydrid, sowie epoxidiertes Polybutadien. Bevorzugte Vernetzer sind Polyamine, insbesondere Polyoxypropylendiamin und Polyoxypropylentriamin. Daneben sind auch Harnstoff, Polyethylenimin sowie Triethylenglykol als Vernetzer geeignet. Die Vernetzungsreaktion kann in Gegenwart des aktiven Agens und/oder in alkoholischer Lösung, z. B. in Dipropylenglykol, bei erhöhter Temperatur erfolgen. Als hydrophile Gruppen wirken insbesondere die von den Polyoxyalkylenpolyaminen stammenden -CRH-O-Gruppen, daneben auch die Maleinsäureanhydrid- und Carboxylgruppen oder Epoxidgruppen bzw. die -NR-CO -Gruppen des vernetzten Polymeren.

Eine andere Klasse von vernetzten Polymeren sind Copolymerisate von monofunktionellen (Meth-)Acrylat-Monomeren, z. B. Hydroxyethylacrylat oder Poly(propylenoxid)(ethylenoxid)monomethacrylat, mit einem polyfunktionellen (Meth-)Acrylat-Monomeren, z. B Ethylenglykoldimethacrylat oder Polyethylenglykol-400- dimethacrylat als Vernetzer. Die Herstellung der vernetzten (Meth-)Acrylat-Polymeren erfolgt durch radikalische Copolymerisation der Monomeren.

Eine typische Zusammensetzung der Ausgangsmaterialien bei der Herstellung der bevorzugten schwammartigen Masse sieht folgendermaßen aus:
Maleinisierte oder epoxidierte Polymere

| | |
|---|---|
| bzw. monofunktionelle (Meth-)Acrylat-Monomere | 10 bis 30 Gew.-% |
| Vernetzer: | 0,2 bis 10 Gew.-% |
| Wasser: | 0,5 bis 20 Gew.-% |
| Flammschutzmittel: | 0 bis 20 Gew.-% |
| Aktives Agens: | Rest |

In beiden Fällen sind die vernetzten Polymeren in der Lage, Flüssigkeiten und Gase, z. B. die aktiven Agentien, zu absorbieren, wobei sich eine Schwammstruktur ausbildet. Das vernetzte Polymere weist ein räumliches Netzwerk mit Poren auf, in dem die flüchtigen Fremdsubstanzen aufgesaugt und absorbiert werden können, so dass das Polymere wie ein Schwamm anquillt. Im angequollenen Zustand besteht das dreidimensionale Netzwerk aus Elementarzellen, die im Mittel ein Volumen von 1 bis 1000 nm³, vorzugsweise von 3 bis 200 nm³, aufweisen.

Das vernetzte Polymere ist erfindungsgemäß mit einem aktiven Agens beladen und bildet mit diesem eine schwammartige Masse. Das aktive Agens wird daraus langsam freigesetzt und kann dann mit in der Anlage vorhandenen übelriechenden Substanzen, z. B. Aminen, Ammoniak und Schwefelverbindungen reagieren, diese reduzieren bzw. maskieren. Die aktiven Agentien sind meist flüssige Aldehyde, Ketone, Alkohole oder Ester, beispielsweise Vanillin, Eugenol, Thymol, Geraniol, Kampferöl, Citroriellol, Linanol, Menthol, Cumarin, Citral, Alpha- Pinen, Nerylacetat, Linalylacetat, Butylhydroxytoluol, C7- bis C12-Aldehyde, Salicylsäurebenzylester und natürliche ölige Essenzen. Neben eigentlichen chemischen Reaktionen, z. B. zwischen Schwefelwasserstoff oder Ammoniak und Aldehyden kann es auch zu Bindungen durch elektrostatische oder van der Waals'sche Kräfte kommen, wodurch die Geruchswahrnehmbarkeit zumindest herabgesetzt wird.

Man kann die aktiven Agentien entweder bei der Herstellung der vernetzten Polymeren durch Kondensation bzw. Polymerisation zusetzen oder das vernetzte Polymere mit den aktiven Agentien tränken und damit anquellen. Das aktive Agens sollte in der schwammartigen Masse in Mengen von 10 bis 90 Gew.-%, vorzugsweise von 40 bis 80 Gew.-% enthalten sein.

Die schwammartige Masse kann neben der Polymermatrix und den aktiven Agentien noch weitere Zusatzstoffe, insbesondere Wasser in Mengen von mindestens 0,1 Gew.%, vorzugsweise von 0,5 bis 20 Gew.% und insbesondere von 1 bis 8 Gew.%, enthalten, ferner 1 bis 20 Gew.-% Flammschutzmittel, wie Zucker, Azodicarbonamid oder Bromverbindungen sowie Pulver zur Verminderung des Verbackens und Sublimationshilfsmittel. Wasser unterstützt die Bildung von Poren und Kanälen bei der Herstellung der vernetzten Polymeren und sorgt dafür, dass die aktiven Agentien leichter in die Poren eindringen können, darin festgehalten werden und gleichmäßig wieder ausdiffundieren.

Wesentlich ist, dass das aktive Agens mit dem zur Anwendung kommenden vernetzten Polymeren und gegebenenfalls dem Gehalt an Wasser so abgestimmt ist, dass es langsam und gleichmäßig aus der schwammartigen Masse freigesetzt wird und seine Wirkung mindestens drei Tage, vorzugsweise mindestens eine Woche und insbesondere mehr als einen Monat lang beibehält. Das freigesetzte aktive Agens kann mit den übelriechenden Substanzen in der Gasphase reagieren. Darüber hinaus ist die schwammartige Masse in der Lage, gasförmige übelriechende Substanzen zu absorbieren und dadurch aus der Luft zu entfernen.

Die schwammartige Masse, welche die aktiven Agentien enthält, kann in Form von Kugeln, Spänen oder Granulat zur Anwendung kommen. Sie wird jedoch bevorzugt in Form von Krümeln, Platten oder Steifen mit einer Dicke von 0,2 bis 5 cm, insbesondere von 0,5 bis 3 cm, eingesetzt, die auf Gitter oder Netze aufgelegt werden. Die schwammartige Masse wird erfindungsgemäß zwischen parallele Platten eingelagert. Die Platten können aus Holz, Metall oder Kunststoff bestehen. Ihre Abmessungen betragen vorzugsweise 5x5 cm bis 100x100 cm, der Abstand der Platten voneinander kann zwischen 2 und 20 cm, insbesondere zwischen 5 und 15 cm liegen. Das Plattenpaar ist allseits offen, so dass der Luftstrom von allen Seiten her durchströmen kann. Die Platten werden vorzugsweise auf senkrecht stehenden Pfosten befestigt, sie können aber auch an Seilen aufgehängt oder einfach auf den Boden aufgelegt werden.

Zwischen diesen parallelen Platten hindurch wird die schwammartige Masse von einem Luftstrom bestrichen, der die aktiven Agentien freisetzt. Dabei wirkt natürlicher Wind als Luftstrom, wobei wahrscheinlich zwischen den parallelen Platten durch den Widerstand der schwammartigen Masse die Luft komprimiert und dadurch der Kontakt zwischen der Luft und den aktiven Agentien verstärkt wird.

Falls dies nicht ausreicht, kann der Luftstrom auch durch ein Gebläse oder einen Ventilator verstärkt werden. Die freigesetzten Agentien vermischen sich mit den aus den übelriechenden Stoffen ausdünstenden Gasen und können mit diesen reagieren. Durch Verstärken des Luftstroms, falls möglich, und durch die Wahl der aktiven Agentien und ihr Zusammenwirken mit den Polymeren und gegebenenfalls dem Wasser kann die Menge der freigesetzten Agentien gesteuert werden und dadurch auch über größere Entfernung zwischen Emissionsstelle und Immissionsstelle hinweg eine ausreichende Geruchsverminderung erreicht werden.

Vorzugsweise werden die parallelen Platten, zwischen die die schwammartige Masse in Netzen oder Gitter eingelagert ist, horizontal auf senkrecht stehenden Pfosten befestigt. Diese Pfosten können am Rand der großflächigen Anlage und/oder in dieser verteilt aufgestellt werden. Beispielsweise werden eine Vielzahl von parallelen Platten, z. B. mehr als fünf, insbesondere zwischen zehn und zweihundert, rings um eine großflächige Anlage herum oder in dieser verteilt angeordnet. Wenn dann ein Wind aus einer Richtung weht (und zwar gleichgültig aus welcher Richtung, werden nicht nur die übelriechenden Substanzen in dieser Richtung von der Oberfläche der Anlage abgetrieben, sondern gleichzeitig setzt der Luftstrom auch die aktiven Agentien aus der schwammartigen Masse frei, die in der selben Richtung abgetrieben werden und mit den übelriechenden Substanzen reagieren.

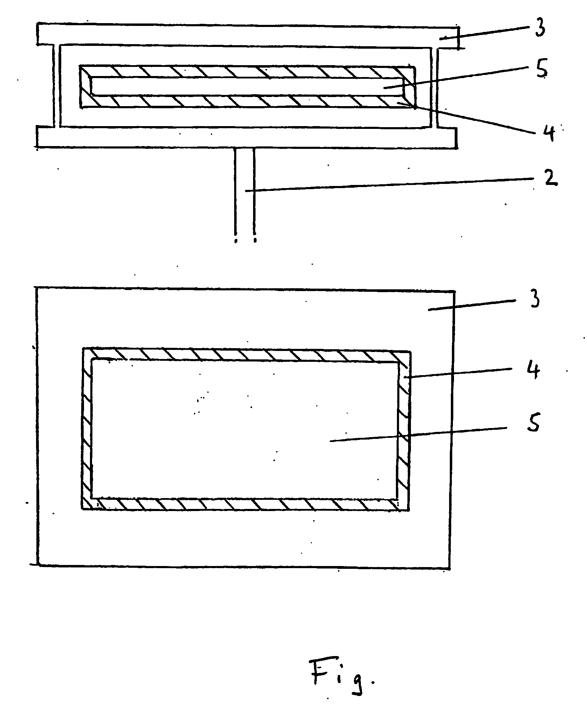

Eine Einrichtung zum Desodorieren von großflächigen Anlagen, bestehend aus einem allseits offenen Paar paralleler Platten, zwischen die eine schwammartige Masse eingelagert ist, welche flüchtige desodorierende Agentien enthält, ist in der Abbildung skizziert. Dabei sind mit 1 die Oberfläche der übelriechenden Stoffe, mit 2 der Pfosten und mit 3 die parallelen Platten bezeichnet, zwischen denen in einem Netz oder Gitter 4 die schwammartige Masse 5 eingelagert ist.

### Beispiel 1

21 g maleinisiertes Polybutadien (Umsetzungsprodukt von flüssigem Polybutadien mit Maleinsäureanhydrid - LITHENE der Fa. Revertex) wurden mit 5 g Wasser und 79 g einer Mischung von öligen Essenzen als aktives Agens bei 45° C vermischt (Mischung A). 94 g des aktiven Agens und 7,5 g Polyoxypropylentriamin (MG 400) wurden vermischt (Mischung B). Die Mischungen A und B wurden zusammengerührt.
Die erhaltene schwammartige Masse wurde in Platten von 2 cm Dicke, 20 cm Breite und 20 cm Länge geschnitten und auf ein Metallnetz aufgelegt. Dieses Netz wurde zwischen zwei parallele, 50 x 50 cm große Kunststoffplatten mit einem Abstand von 10 cm eingebracht. 16 derartiger Platten wurden ringsum an der schrägen Einfassung eines 100 m² großen Klärbeckens aufgelegt.

### Beispiel 2

In einem geschlossenen Gefäß wurden 4 g Poly(propylenoxid)(ethylenoxid)- Monomethacrylat mit 0,5 g Polyethlenglykol-400-Dimethacrylat vermischt und 100 Mikroliter 30%-iges Wasserstoffperoxid wurden injiziert. Die Mischung wurde 30 min. lang in einem Ultraschallbad evakuiert. Dann wurde die Mischung in ein offenes, flaches Gefäß geleert und dort bei 10° C 5 min. lang mit UV- Licht (290 bis 400 nm, 40 mW/cm²) in einem Abstand von 5 cm bestrahlt.
Das erhaltene vernetzte Polymere wurde mit 3g Salicylsäurebenzylester als aktivem Agens getränkt. Die erhaltene schwammartige Masse wurde in die erfindungsgemäße Einrichtung eingebracht. Einhundert solcher Einrichtungen wurden auf Pfosten befestigt und in einer Mülldeponie verteilt aufgestellt.

### Beispiel 3

15 g epoxidiertes Polybutadien (POLY BD von Atofina), 3 g Polyoxypropylen-diamin (Jeffamine D 400 von Huntsman) und 8 g Wasser wurden mit 70 g eines flüssigen Aldehyds vermischt, entgast und gerührt. Die Masse wurde 5 mm dick auf einen Träger aufgetragen und mittels eines 80°C heißen Luftstroms polymerisiert.

Drei Lagen des entstandenen Blattes wurden übereinandergelegt und in 20 mm breite Streifen geschnitten. Diese wurden auf Netze aufgelegt, die zwischen zwei Platten eingebracht wurden. Zehn derartiger Platten wurden in einer Kompostieranlage aufgestellt.

## Patentansprüche

1. Verfahren zum Desodorieren von großflächigen Anlagen, ausgewählt aus Klärbecken, Kläranlagen, Kompostieranlagen, Deponien, Müllsortieranlagen, Abwasserstapelbecken, Hafenbecken und offene Kanäle, in denen übelriechende feste oder flüssige Stoffe offen gelagert sind, durch Behandeln der verunreinigten Luft über der Oberfläche der übelriechenden Stoffe mit aktiven Agenzien, die mit den in der Luft enthaltenen übelriechenden Substanzen reagieren, wobei die aktiven Agenzien in einer Matrix aus einem vernetzten, hydrophile Gruppen enthaltenden Polymer verteilt sind, und mit diesem zusammen eine schwammartige Masse bilden, aus der die aktiven Agenzien langsam freigesetzt werden und verdunsten, und wobei die schwammartige Masse zwischen zwei parallelen Platten eingelagert ist, wobei die schwammartige Masse in einem Netz oder Gitter zwischen den parallelen Platten eingelagert ist, die in einer Vielzahl über der Oberfläche der übel riechenden Stoffe oder (ringsum) am Rand der großflächigen Anlage angebracht sind, wobei das Plattenpaar allseits offen ist und die schwammartige Masse in Form von Platten oder Streifen mit einer Dicke von 0,2 bis 3 cm vorliegt und der Abstand der parallelen Platten voneinander 5 bis 20 cm beträgt, so dass ein Luftstrom von natürlichem Wind zwischen den parallelen Platten, hindurch die schwammartige Masse bestreicht und die aktiven Agenzien freisetzt.

2. Verfahren nach Anspruch 1, **dadurch gekennzeichnet, dass** das vernetzte Polymer_ein Kondensationsprodukt aus einem maleinisierten oder epoxidierten Polymeren und einem Polyamin als Vernetzer ist.

3. Verfahren nach Anspruch 1, **dadurch gekennzeichnet, dass** das vernetzte Polymer_ein Copolymerisat aus einem monofunktionellen (Meth-)AcrylatMonomeren und einem polyfunktionellen (Meth-)Acrylat-Monomeren als Vernetzer ist.

4. Verfahren nach Anspruch 1, **dadurch gekennzeichnet, dass** die aktiven Agenzien über einen Zeitraum von mindestens drei Tagen hinweg langsam und gleichmäßig aus der schwammartigen Masse freigesetzt werden.

5. Verfahren nach Anspruch 1, **dadurch gekennzeichnet, dass** die aktiven Agenzien in Mengen von 10 bis 90 Gew.% in der schwammartigen Masse enthalten sind.

6. Verfahren nach Anspruch 1, **dadurch gekennzeichnet, dass** die aktiven Agenzien Aldehyde, Ketone, Alkohole, Ester oder natürliche ölige Essenzen sind.

7. Verfahren nach Anspruch 1, **dadurch gekennzeichnet, dass** die schwammartige Masse mindestens 0,1 Gew.-%, vorzugsweise 1 bis 8 Gew.-% Wasser enthält.

8. Verfahren nach Anspruch 1, **dadurch gekennzeichnet, dass** die schwammartige Masse zusätzlich Flammschutzmittel, Sublimationshilfsmittel und/oder Pulver zur Verhinderung des Verbackens enthält.

## Claims

1. Method for deodorizing large-scale facilities selected from settling basins, sewage treatment plants, composting plants, landfills, refuse sorting plants, wastewater holding basins, docks and open channels in which foul-smelling solid or liquid substances are stored in the open, by treating the contaminated air over the surface of the foul-smelling substances with active agents which react with the foul-smelling substances present in the air, wherein the active agents are distributed in a matrix made of a crosslinked polymer containing hydrophilic groups, and, together with this, form a sponge-like mass, from which the active agents are released slowly and vaporize, and wherein the sponge-like mass is situated between two parallel plates, wherein the sponge-like mass is situated in a net or grating between the parallel plates which are mounted in a multiplicity over the surface of the foul-smelling substances or are mounted all around at the rim of the large-scale facility, wherein the plate pair is open on all sides and the sponge-like mass is present in the form of plates or strips having a thickness of 0.2 to 3 cm and the distance of the parallel plates from one another is 5 to 20 cm, in such a manner that an airstream of natural wind sweeps through the sponge-like mass between the parallel plates and releases the active agents.

2. Method according to Claim 1, **characterized in that** the crosslinked polymer is a condensation product of a maleinized or epoxidized polymer and a polyamine as crosslinker.

3. Method according to Claim 1, **characterized in that** the crosslinked polymer is a copolymer of a monofunctional (meth)acrylate monomer and a polyfunctional (meth)acrylate monomer as crosslinker.

4. Method according to Claim 1, **characterized in that** the active agents are released from the sponge-like mass slowly and uniformly over a period of at least three days.

5. Method according to Claim 1, **characterized in that** the active agents are present in the sponge-like mass in amounts of 10 to 90% by weight.

6. Method according to Claim 1, **characterized in that** the active agents are aldehydes, ketones, alcohols, esters or natural oily essences.

7. Method according to Claim 1, **characterized in that** the sponge-like mass contains at least 0.1% by weight, preferably 1 to 8% by weight, of water.

8. Method according to Claim 1, **characterized in that** the sponge-like mass additionally contains flame retardants, sublimation aids and/or powders for preventing caking.

## Revendications

1. Procédé de désodorisation d'installations de grande surface, sélectionnées parmi des bassins de décantation, des stations d'épuration, des stations de compostage, des décharges, des centres de tri des ordures, des réservoirs d'accumulation des eaux usées, des docks et des canaux ouverts, dans lesquelles sont stockés à découvert des substances solides ou liquides malodorantes, par traitement de l'air pollué au-dessus de la surface des substances malodorantes avec des agents actifs qui réagissent avec les substances malodorantes contenues dans l'air, où les agents actifs sont répartis dans une matrice d'un polymère réticulé contenant des groupes hydrophiles, et forment avec celui-ci une masse spongieuse à partir de laquelle les agents actifs sont lentement libérés et volatilisés, et où la masse spongieuse est incorporée entre deux plaques parallèles, où la masse spongieuse est incorporée dans un réseau ou une grille entre les plaques parallèles, qui sont appliquées selon un certain nombre au-dessus de la surface des substances malodorantes ou à l'entour au niveau du bord de l'installation de grande surface, où la paire de plaques est ouverte de tous côtés et la masse spongieuse se présente sous la forme de plaques ou de bandes d'une épaisseur comprise entre 0,2 et 3 cm et l'écart entre les plaques parallèles est compris entre 5 et 20 cm, si bien qu'un courant d'air de vent naturel entre les plaques parallèles traverse la masse spongieuse et libère les agents actifs.

2. Procédé selon la revendication 1, **caractérisé en ce que** le polymère réticulé est un produit de condensation d'un polymère maléinisé ou époxydé et d'une polyamine en tant qu'agent de réticulation.

3. Procédé selon la revendication 1, **caractérisé en ce que** le polymère réticulé est un produit de copolymérisation d'un monomère de (méth)acrylate monofonctionnel et d'un monomère de (méth)acrylate polyfonctionnel en tant qu'agent de réticulation.

4. Procédé selon la revendication 1, **caractérisé en ce que** les agents actifs sont libérés lentement et régulièrement de la masse spongieuse sur une durée au moins égale à trois jours.

5. Procédé selon la revendication 1, **caractérisé en ce que** les agents actifs sont contenus dans la masse spongieuse dans des quantités comprises entre 10 et 90 % en poids.

6. Procédé selon la revendication 1, **caractérisé en ce que** les agents actifs sont des aldéhydes, des cétones, des alcools, des esters ou des essences huileuses naturelles.

7. Procédé selon la revendication 1, **caractérisé en ce que** la masse spongieuse contient au moins 0,1 % en poids, de préférence 1 à 8 % en poids, d'eau.

8. Procédé selon la revendication 1, **caractérisé en ce que** la masse spongieuse contient en outre des agents ignifugeants, des auxiliaires de sublimation et/ou de la poudre destinée à empêcher l'agrégation.
